# EUROPÄISCHE PATENTSCHRIFT

(11) **EP 1 556 481 B1**
(45) Veröffentlichungstag und Bekanntmachung des Hinweises auf die Patenterteilung: **18.10.2006**
(21) Anmeldenummer: 03708252.6
(22) Anmeldetag: 18.03.2003
(51) Int. Cl.: C12N 5/06

(54) **VERFAHREN ZUR AKTIVIERUNG VON DENDRITISCHEN ZELLEN**
METHOD FOR ACTIVATING DENDRITIC CELLS
PROCEDE D'ACTIVATION DE CELLULES DENDRITIQUES

(30) Priorität: 26.03.2002 DE 10213493
(43) Veröffentlichungstag der Anmeldung: 27.07.2005
(73) Patentinhaber: Grabbe, Stephan, 48149 Münster (DE)
(72) Erfinder: Grabbe, Stephan, 48149 Münster (DE)
(74) Vertreter: Gesthuysen, von Rohr & Eggert
(86) Internationale Anmeldenummer: PCT/EP2003/002807
(87) Internationale Veröffentlichungsnummer: WO 2003/080815

(56) Entgegenhaltungen:
- WO-A-00/20581
- WO-A-01/51617
- SALLUSTO F ET AL: "EFFICIENT PRESENTATION OF SOLUBLE ANTIGEN BY CULTURED HUMAN DENDRITIC CELLS IS MAINTAINED BY GRANULOCYTE/MACROPHAGE COLONY-STIMULATING FACTOR PLUS INTERLEUKIN 4 AND DOWNREGULATED BY TUMOR NECROSIS FACTOR ALPHA" JOURNAL OF EXPERIMENTAL MEDICINE, TOKYO, JP, Bd. 179, Nr. 4, 1. April 1994 (1994-04-01), Seiten 1109-1118, XP000611720 ISSN: 0022-1007

## Beschreibung

Gegenstand der vorliegenden Erfindung ist ein Verfahren, mit dessen Hilfe dendritische Zellen aktiviert werden können. Die Aktivierung der dendritischen Zellen erfolgt durch Zugabe von Überständen aus aktivierten Thrombozyten.

lmmunantworten wurden von der Evolution so entwickelt, dass sie sich im wesentlichen gegen in den Körper eindringende mikrobielle Erreger richten. Im Prinzip kann man zwei Arten von Immunantworten unterscheiden:
1) Antigen-unspezifische Immunantworten. Zu diesen gehören z. B. Entzündungsreaktionen aller Art, die Aktivierung von keimabtötenden Abwehrzellen, oder die Freisetzung von direkt antimikrobiellen Substanzen.
2) Antigen-spezifische Immunantworten. Diese sind durch Lymphozyten vermittelt und richten sich gegen eine bestimmte Zielstruktur (das "Antigen") auf der Oberfläche des jeweiligen Erregers. Prinzip dieser antigenspezifischen Immunantworten ist es, dass ruhende Lymphozyten zunächst durch Kontakt mit antigen-präsentierenden Zellen aktiviert werden müssen ("Sensibitisierung" oder "Priming"). Dies führt zur Bildung von sog. Gedächtniszellen, die bei erneutem Kontakt mit dem Erreger dann wesentlich schneller und effizienter eine Immunantwort auslösen können. Die de novo-Aktivierung ruhender T-Zellen ist das Prinzip jeder Impfung.

In den letzten Jahren stellte sich heraus, dass der Zelltyp des Organismus, dessen Aufgabe es ist, ruhende T-Zellen zu aktivieren, die dendritische Zelle ist. Bei den dendritischen Zellen handelt es sich um die potentesten antigen-präsentierenden Zellen. Diese müssen aber aktiviert werden, bevor sie die Immunantwort initiieren können. Die Oberfläche der dendritischen Zellen ist ungewöhnlich und weist schleierartige Fortsätze auf. Charakteristisch ist das Vorhandensein der Oberflächenmarker CD83, CD86⁺ oder HLA-DR⁺.

Aus dem Stand der Technik sind verschiedene Verfahren bekannt, wie reife dendritische Zellen bereitgestellt werden können. Die EP 0 922 758 beschreibt ein in vitro-Verfahren zur Herstellung von reifen dendritischen Zellen aus unreifen Vorläuferzellen, die aus pluripotenten Zellen herstammen, wobei die unreifen dendritischen Zellen mit einem Reifungsfaktor inkubiert werden. Bei dem Reifungsfaktor handelt es sich um ein Zytokin, insbesondere Interferon-α, IL-1β, IL-6 und TNFα.

In der WO 98/06823 wird ein Verfahren zur Herstellung von dendritischen Zellen in einer Zytokin-freien Kultur beschrieben. Bei diesem Verfahren werden mononukleäre Zellen aus peripherem Blut, Nabelschnurblut oder Knochenmark in Gegenwart eines Reifungsmittels angezogen. Bei diesen Mitteln kann es sich um Calciumionophoren, Theophyllin, Prostaglandin E1, Dibutyryl cyclisches AMP, Vitamin D3, Vitamin E, Retinolsäure und Fettsäuren handeln.

Auch Verfahren zur Herstellung von dendritischen Zellen in serumfreier Kultur sind aus der WO 98/06826 bekannt.

Art und Umfang der T-Zell-Aktivierung hängt entscheidend von der Antigen-Präsentation ab. Dendritische Zellen (DC) sind die wichtigsten antigen-präsentierenden Zellen des Organismus und somit von zentraler Bedeutung bei der Entstehung von zellvermittelten Immunantworten. Die Aufgabe von DC ist es, fremde und körpereigene Substanzen ("Antigene") dem Immunsystem so darzubieten, dass dieses die Substanzen erkennen kann und eine nachfolgende Immunantwort induziert wird. Art und Ausmaß dieser Immunantwort hängt in entscheidendem Maße von dem Aktivitätszustand der antigen-präsentierenden Zellen ab. Neben den DC gibt es noch eine Reihe anderer Körperzellen, die ebenfalls Antigen präsentieren können und die prinzipiell ebenfalls in der Lage sind, Immunantworten auszulösen. DC können dieses jedoch wesentlich effizienter als alle anderen bekannten antigen-präsentierenden Zellen und nach gegenwärtigem Kenntnisstand sind DC die einzigen Zellen, die eine T-Zell vermittelte Immunantwort neu entstehen lassen können. Andere antigen-präsentierende Zellen (APC) können dieses nicht, im Gegenteil führt die Präsentation des Antigens durch andere APC in der Regel nicht nur zu keiner Aktivierung der T-Zellen, sondern sogar zu spezifischer Immuntoleranz, d.h. zur gezielten Unterdrückung einer spezifischen Immunantwort. Auch die Antigen-Präsentation durch dendritische Zellen führt nicht notwendigerweise zur Immunaktivierung. Art und Ausmaß der Antigen-Präsentation hängen in zentraler Weise vom jeweiligen Aktivierungszustand der dendritischen Zelle ab. Nur optimal aktivierte dendritische Zellen sind in der Lage, wie oben beschrieben native T-Zellen zu aktivieren, während ruhende, nicht aktivierte, dendritische Zellen Antigen ebenfalls in tolerogener Weise präsentieren.

Durch ihre einzigartige Fähigkeit, ruhende T-Zellen zu aktivieren und somit primäre Immunantworten induzieren zu können, sind dendritische Zellen zu einem attraktiven Therapeutikum für Impfungen aller Art avanciert. Ziel von Impfungen aller Art ist es erstens eine primäre Immunantwort neu zu erzeugen, zweitens eine ausreichend starke Immunantwort zu induzieren und drittens die Art der Immunantwort zu kontrollieren, vor allem im Hinblick auf die Entstehung von T-Helfer-1 bzw. T-Helfer-2 Immunantworten.

Um dendritische Zellen therapeutisch einzusetzen, ist es somit zwingend, diese in einen adäquaten Funktionszustand zu versetzen. Die aktivierten dendritischen Zellen werden bei den verschiedensten Impfungen verwendet. Ein bevorzugtes Anwendungsgebiet ist die Tumorvakzinierung, bei der therapeutisch eine Immunantwort gegen die im Körper vorhandenen Tumore erzeugt wird.

Aus dem Stand der Technik sind eine Reihe von Faktoren bekannt geworden, die zur Aktivierung von dendritischen Zellen in der Lage sind. Zu diesen gehören eine Reihe von bakteriellen Produkten (LPS, CpG, und andere bakterielle Polysaccharide und Nukleinsäuren), pro-inflammatorische Zytokine (IL-1, IL-6, TNFα, GM-CSF, Heat Shock Proteine, u.a.), sowie vorwiegend von Leukozyten exprimierte Moleküle der TNF/TNF-R Molekülfamilie (CD40L, RANKL). Zudem sind eine Reihe von Faktoren identifiziert worden, die die Aktivierung dendritischer Zellen dahingehend modulieren, dass durch diese dann vorwiegend T-Helfer-1 bzw. T-Helfer-2 Immunantworten induziert werden. Zu den T-Helfer-1 induzierenden Faktoren gehören unter anderem IFNγ, IL-12, IL-18, bestimmte CpGs, sowie CD40L. Zu den T-Helfer-2 induzierenden Faktoren gehören PGE₂ und IL-10. Es ist bislang jedoch noch unklar, ob die verschiedenen DC-Aktivatoren unterschiedliche Arten von Immunantworten nach Injektion der DC in Personen zum Zwecke der Immuntherapie hervorrufen. Dendritische Zellen werden in Abwesenheit von fremden Substanzen durch endogene Signale aktiviert, die sie von Zellen erhalten, die gestreßt werden, viral infiziert werden oder abgetötet werden (Gallucci et al., Nature Medicine, Vol. 5, Nr. 11, November 1999, S. 1249-1255).

Die WO 98/01538 offenbart ein Verfahren der Aktivierung von dendritischen Zellen, bei dem die dendritischen Zellen zunächst mit einem Antigen in Kontakt gebracht werden und dann mit einem Protein, das an den CD40-Rezeptor binden kann, dem sogenannten CD40-Ligand (CD40L) aktiviert werden.

Gegenstand der vorliegenden Erfindung ist ein in vitro Verfahren zur Aktivierung von dendritischen Zellen, wobei die Aktivierung dadurch erreicht wird, daß die nicht aktivierten dendritischen Zellen mit einem Überstand von stimulierten Thrombozyten in Kontakt gebracht werden.

Die in dem Verfahren eingesetzten dendritischen Zellen werden aus geeigneten Vorläuferzellen gewonnen. Bei den Vorläuferzellen kann es sich um embryonale Stammzellen handeln oder um Stammzellen, die noch die Fähigkeit zur Differenzierung besitzen. Derartige Vorläuferzellen können beispielsweise aus Knochenmarkaspirat oder Nabelschnurblut gewonnen werden. Üblicherweise werden aber dendritische Zellen aus Monozyten des peripheren Blutes gewonnen. Die Differenzierung dieser Vorläuferzellen zu dendritischen Zellen erfolgt in der Regel dadurch, daß die Vorläuferzellen in Gegenwart von geeigneten Wachstumsfaktoren bzw. einer Kombination dieser Wachstumsfaktoren kultiviert werden. Bevorzugt wird hierbei eine Kombination der Wachstumsfaktoren GM-CSF und IL-4 verwendet.

Die Aktivierung der dendritischen Zellen erfolgt erfindungsgemäß dadurch, daß ein Kulturüberstand aus stimulierten Thrombozyten verwendet wird. Bei den Thrombozyten handelt es sich um eine Untergruppe der weißen Blutkörperchen, die aus peripherem Blut oder Leukapherese-Material isoliert werden können. Eine bevorzugte Form der Auftrennung erfolgt dadurch, daß aus Leukapherese-Material plättchenreiches Plasma hergestellt wird.

Es hat sich herausgestellt, daß die Aktivierung der dendritischen Zellen dann besonders effektiv ist, wenn die Thrombozyten, deren Überstand gewonnen wurde, vorher aktiviert wurden. Die Aktivierung kann dadurch erfolgen, daß übliche Thrombozytenaktivatoren mit den Thrombozyten in Kontakt gebracht werden. In einer besonders bevorzugten Ausführungsform werden die Thrombozyten mit Thrombin oder einem ThrombinSpaltprodukt in Kontakt gebracht.

Grundsätzlich können die dendritischen Zellen von geeigneten Spendern herstammen und die Thrombozyten können auch von anderen Spendern stammen. Es ist also durchaus denkbar, daß die dendritischen Zellen aus Knochenmarkszellen, Nabelschnurblut oder Monocyten gewonnen werden und die Thrombozyten beispielsweise aus Blutspenden, die auch gepoolt sein können, gewonnen werden.

In einer besonders bevorzugten Ausführungsform handelt es sich aber um ein autologes System, d.h. die dendritischen Zellen stammen ebenso wie die Thrombozyten von demjenigen Patienten, dem die aktivierten dendritischen Zellen zur Immunisierung wieder verabreicht werden. Es ist also möglich, einem Patienten vor der Behandlung Blut abzunehmen und hieraus separat die Vorläuferzellen für die dendritischen Zellen und die Thrombozyten zu gewinnen, anschließend die Thrombozyten zu aktivieren und den Überstand dazu zu verwenden, um damit die dendritischen Zellen zu aktivieren.

Als endogener Stimulus, der dendritische Zellen wirkungsvoll aktivieren kann, ist der an den Rezeptor CD40 bindende Ligand CD40L (CD40 Ligand) bekannt (The Journal of Immunology, 1999, 162: 168-175). Im Rahmen der vorliegenden Erfindung wurde daher als Parameter für die Qualität und die Konzentriertheit des für die Aktivierung von dendritischen Zellen verwendeten konditionierten Mediums der Gehalt an CD40L gewählt. Die Konzentration an CD40L wird mit Hilfe eines kommerziell erhältlichen ELISA (Fa. Chemikon) bestimmt.

Für die Herstellung der dendritischen Zellen (DC) wird zunächst eine auf Gewinnung von Monozyten optimierte Leukapherese nach Standardverfahren durchgeführt. Vorzugsweise erfolgt diese ohne vorherige Stammzell-Mobilisierung und ohne Elutriation. Alternativ können jedoch auch andere, zur Gewinnung von Monozyten/DC geeignete Leukaphereseverfahren verwendet werden (z.B. mit vorheriger in vivo Mobilisierung der Präkursorzellen durch Gabe von Wachstumsfaktoren, z.B. mit Elutriation zur Aufreinigung der Monozyten). Die gewonnenen mononukleären Zellen des periphen Bluts (PBMC) dienen als Ausgangsmaterial für die Herstellung der DC.

Die bevorzugte Herstellungsmethode für DC besteht in der Kultur der plastik-adhärenten PBMC in der Gegenwart von 1000 U/ml GM-CSF und 1000 U/ml IL-4 für 6 Tage in RPMI1640 und 1 % autologem Plasma. Neben DC, die aus Monozyten nach einem der etablierten Verfahren kultiviert bzw. isoliert werden, können jedoch auch auf andere Art gewonnene DC verwendet werden (z.B. DC-Kultur aus PBMC mit oder ohne vorherige Anreicherung der DC-Präkursoren, z.B. durch Adhärenz an Plastik oder andere Adhärenzmedien, durch Verwendung monoklonaler Antikörper und nachfolgender positiver Selektion der DC-Präkursoren oder negativer Depletion der kontaminierenden Zellen, DC-Kultur aus CD14⁺ Monozyten, CD34⁺ Stammzellen oder anderen DC-Präkursoren, direkte ex vivo Isolation von DC aus dem peripheren Blut).

Die so gewonnenen "unreifen" DC dienen als Ausgangssubstanz für das erfindungsgemäße Verfahren der DC-Aktivierung.

Die Aktivierung der dendritischen Zellen erfolgt durch Zugabe eines Überstandes (konditionierten Mediums) aus Thrombozyten. Bevorzugt wird hierfür autologes plättchenreiches Plasma (PRP) nach Standardverfahren hergestellt bzw. entsteht als Nebenprodukt bei der Leukapherese. Alternativ zu autologem PRP kann auch allogenes PRP verwendet werden, wobei allerdings darauf geachtet werden muß, daß unerwünschte virale Kontaminationen vermieden werden.

Die im PRP enthaltenen Thrombozyten (TZ) werden nun bevorzugt stimuliert, und es wird ein konditioniertes Medium (Überstand) hergestellt, welches zur Aktivierung der DC verwendet wird.

Dieser Kulturüberstand (konditioniertes Medium) wird in einer bevorzugten Ausführungsform wie folgt hergestellt:

Das PRP wird bei RT unter ständiger langsamer Agitation (Kipp-/Schwenkplatte) in sterilen, verschlossenen Beuteln gelagert. Der PRP-Beutel wird geöffnet und der Inhalt in 50 ml Falcon-Röhrchen umgefüllt. TZ des PRP werden gezählt. Die TZ werden anschliessend zentrifugiert und der Überstand wird vorsichtig abpipettiert. Die TZ werden mit PBS auf eine TZ-Konzentration von 1 × 10¹⁰/ml (1 × 10³/ml - 1 × 10¹²/ml) eingestellt. Die TZ werden nun für 3-5 h mit 2,0 U/ml (0.02 - 200 U/ml) humanem Thrombin inkubiert (bei 37°C). Die Reaktionsgefäße werden anschliessend 5 × bei -80°C oder im Einfrierapparat eingefroren und anschließend wieder aufgetaut. Die Reaktionsgefäße werden anschliessend für 15-30 min bei ≥2000 x g zentrifugiert, Überstände werden abpipettiert und auf Centri-Plus™-Säule zwecks Konzentrierung gegeben (Centri-Plus YM 10, cut-off 10Kda). Die TZ-Überstände werden dann bei 3000 x g über Nacht (oder >4 Std.) bei 4°C zentrifugiert. Das Retentat wird abpipettiert und als Substrat für die DC-Aktivierung verwendt.

Eine bevorzugte Art der Thrombozyten-Aktivierung ist die Verwendung von Thrombin bzw. einem Thrombinspaltprodukt (TRAP). Es können jedoch auch andere Verfahren der Thrombozytenaktivierung verwendet werden (z.B. die Zugabe von ADP, PMA, Prostaglandine und deren Analoga, andere Gerinnungsfaktoren und deren Analoga, die Ligation von Thrombozyten-Oberflächenrezeptoren durch Antikörper oder Rezeptorbindende Moleküle, mechanische/thermische Aktivierung, etc.). Bevorzugt ist auch die Kombination mehrerer Aktivierungsschritte. Auch nichtaktivierte TZ können verwendet werden, indem ein Proteinextrakt nach Zytolyse der TZ hergestellt wird. Auch eine TZ-Aktivierung während der Ko-Inkubation mit DC ist möglich. In diesem Fall werden Vorläuferzellen bzw. nicht aktivierte DC, TZ und Aktivierungsmittel gleichzeitig inkubiert. Bevorzugte Art der Konzentrierung des TZ-Überstands ist die Verwendung von CentriPlus-Gefäßen, andere Konzentrierungsverfahren können jedoch ebenfalls Verwendung finden. Bevorzugte Art der Lyse der TZ ist repetitives Einfrieren und Auftauen, andere Arten der Zelldisruption können jedoch ebenfalls verwendet werden.

Der TZ-Überstand enthält CD40L, welches zur Abschätzung der Qualität des konditionierten Mediums gemessen wird. Daneben enthält das konditionierte Medium jedoch noch andere Faktoren, die zur DC-Aktivierung beitragen.

Bevorzugt werden DC während der Inkubation in konditioniertem Medium zusätzlich mit 0-5000 U/ml GM-CSF und 0-5000 U/ml IL4 oder IL13 (bevorzugt: 1000 U/ml GM-CSF und 1000 U/ml IL4) kultiviert. Zusätzlich wird 10 % v/v (0.5 - 50%) TZ-Überstand in die Kultur gegeben. Andere DC-Aktivierungsfaktoren, wie z.B. Poly I:C, bakterielle Produke (LPS, Bakterienextrakte, bakt. RNA/DNA, IFNγ, IL12+IL18 etc ) können zusätzlich verwendet werden. Die Aktivierung der DC erfolgt für 4-72 h (bevorzugt: 36h). Anschliessend werden die DC 3 × mit PBS (Ca⁺⁺/Mg⁺⁺-frei) gewaschen und für die jeweilige Applikation verwendet.

Der erfindungsgemäß eingesetzte TZ-Überstand kann neben der Aktivierung von DC auch für andere Zwecke verwendet werden. Zu diesen gehören:
- B Zell-Aktivierung
- Mø-Aktivierung
- Endothelzell-Aktivierung
Neben der in vitro Kultur kann der TZ-Überstand auch zur direkten Applikation in vivo mit dem Ziel der selektiven Aktivierung bestimmter Zellpopulationen Verwendung finden. Mögliche andere Applikationsweisen sind u.a.:
- Injektion i.v. oder s.c./i.d.
- Inhalation.

Die nachfolgenden Beispiele sollen die vorliegende Erfindung näher erläutern.

Der zu DC-Aktivierung generierte TZ-Überstand wird bevorzugt wie oben beschrieben hergestellt. Um den Grad der TZ-Aktivierung zu bestimmen, wird der Gehalt an CD40L im TZ-Überstand bestimmt. Mit Hilfe dieses Parameters wurde die Aktivierung des TZ-Überstands bestimmt.

### Beispiel 1

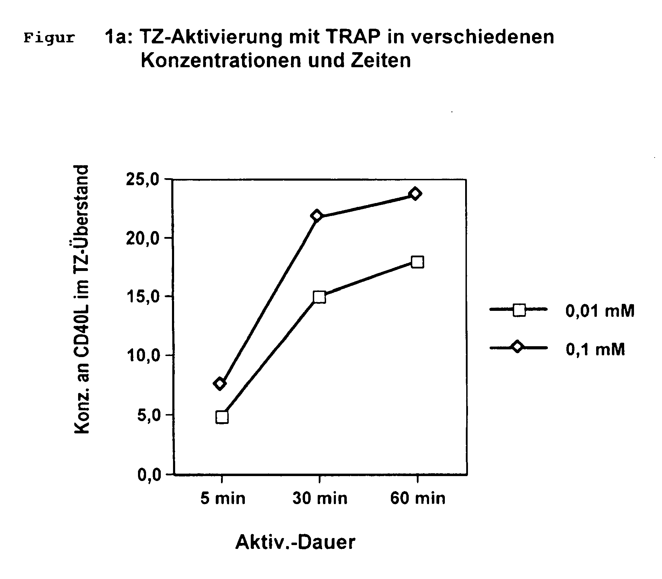

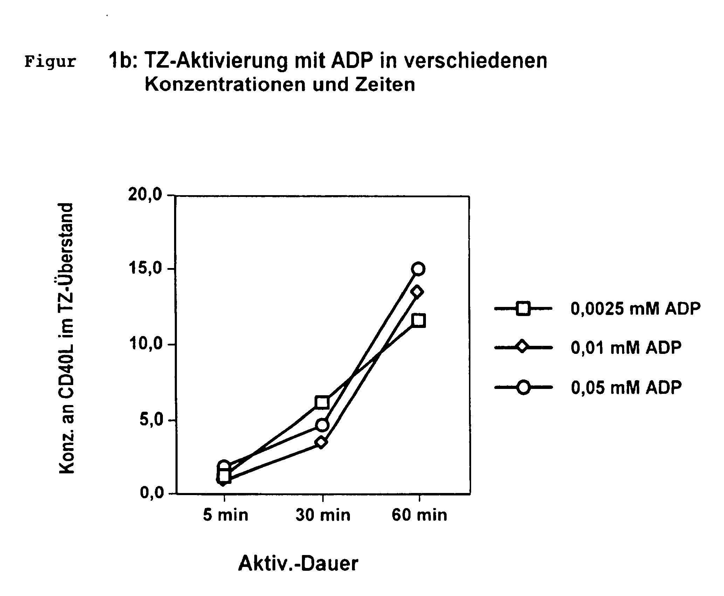

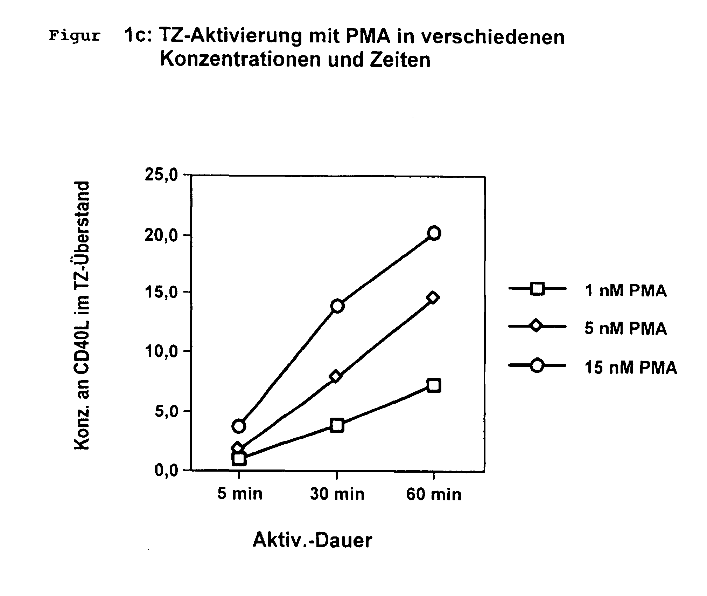

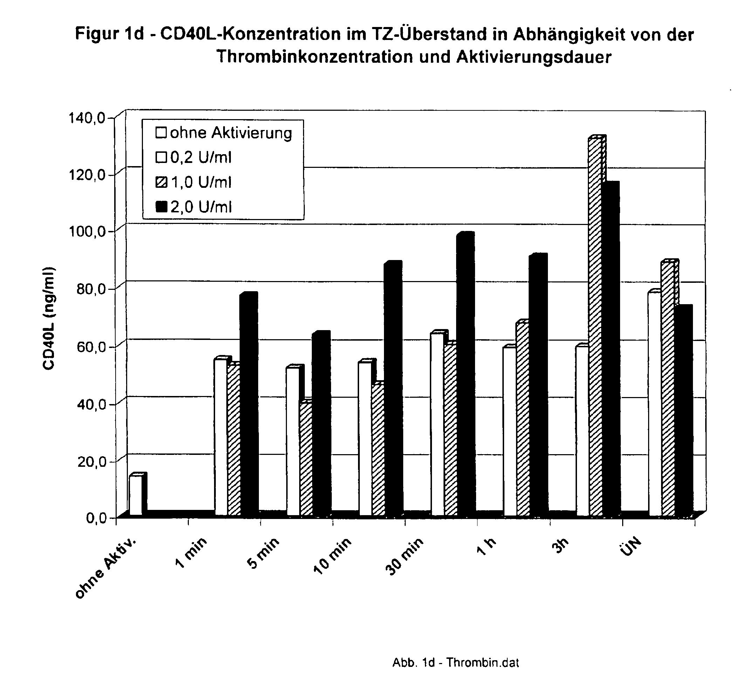

### Vergleich verschiedener TZ-Aktivatoren (Figur 1)

Der zu DC-Aktivierug generierte TZ-Überstand wurde gemäß bevorzugter Ausführungsform hergestellt. Es wurden aber verschiedene Komponenten zur Thrombozytenaktivierung verwendet. Als TZ-Aktivatoren wurden verschiedene Konzentrationen von TRAP (Thrombin-Rezeptor-assoziiertes Protein), ADP (Adenosin-Diphosphat), PMA (Phorbol-Myristat-Acetat) und Thrombin miteinander verglichen und die Konzentration an CD40L im TZ-Überstand gemessen. Es zeigt sich, dass Thrombin eindeutig die höchste Konzentration von CD40L im TZ-Überstand induziert.

Die Ergebnisse der Versuche sind in Figuren 1a -1d dargestellt.
Figur 1a zeigt die Aktivierung der Thrombozyten durch Verabreichung von 0,1 mM bzw. 0,01 mM TRAP (Thrombin-Rezeptor-assoziiertes Protein) gemessen.
Figur 1b zeigt die Aktivierung von Thrombozyten durch Zugabe von 0,0025, 0,01 und 0,05 mM ADP (Adenosin-Ddiphosphat).
Figur 1c zeigt die Thrombozytenaktivierung mit 1 nM, 5 nM und 15 nM PMA (Phorbol-Myristat-Acetat).
Figur 1d zeigt die Aktivierung von Thrombozyten mit dem erfindungsgemäß bevorzugten Aktivator-Thrombin. Gezeigt wird die Abhängigkeit von Thrombinkonzentration und Dauer der Aktivierung. Besonders bevorzugt ist eine Thrombinkonzentration von etwa 1 U/ml und eine Aktivierungsdauer von etwa 3 Stunden.

### Beispiel 2

### Vergleich verschiedener Thrombin- und TZ-Konzentrationen

Der zu DC-Aktivierung generierte TZ-Überstand wurde wie oben beschrieben hergestellt. Abweichend zu dieser wurden die Zelldichte und die Konzentrationen von Thrombin variiert. Der Gehalt an CD40L wurde als Parameter der Qualität des TZ-Überstands bestimmt. Es zeigt sich, dass der CD40L-Gehalt des TZ-Überstands proportional zur eingesetzten TZ-Konzentration zunimmt und dass die TZ-Stimulation mit 2 U/ml Thrombin eine höhere CD40L Ausbeute ergibt als mit 1 U/ml.

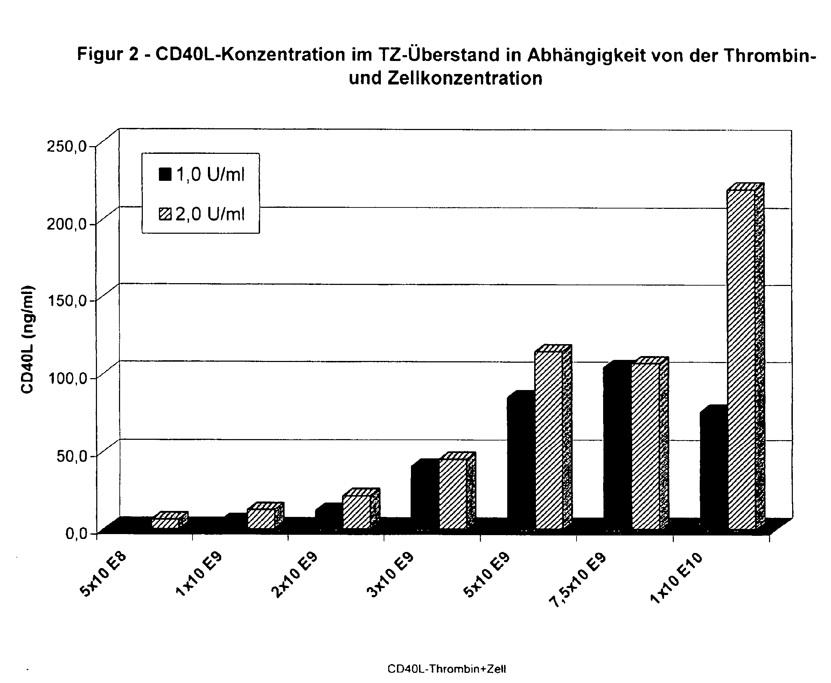

Die Ergebnisse dieses Versuches sind in Figur 2 dargestellt. Die Versuche wurden einmal mit 1 U/ml Thrombin (schwarze Säulen) und einmal mit 2,0 U/ml Thrombin (schraffierte Säulen) durchgeführt. Die Angaben auf der Abszisse geben die TZ-Konzentration an, aus der die Überstände hergestellt wurden (E = Exponent, z.B. E9 = 10⁹).

### Beispiel 3

### CD40L-Gehalt des TZ-Überstands in Abhängigkeit von der Thrombinkonzentration, der Inkubationsdauer und des Gefrierauftauvorganges (TZ-Lyse)

Der zu DC-Aktivierung generierte TZ-Überstand wurde wie oben beschrieben hergestellt. Abweichend zu von der bevorzugten Methode wurden die Aktivierungsdauer und die Konzentrationen von Thrombin variiert. Zusätzlich wurde getestet, ob eine Lyse der Thrombozyten mittels Einfrieren und Auftauen eine weitere Erhöhung der CD40L-Konzentration im Überstand bewirkt. Es zeigt sich, dass die CD40L-Konzentration im TZ-Überstand nach 3-stündiger (in anderen Experimenten: 5-stündiger) Aktivierung mit Thrombin und nachfolgender TZ-Lyse am höchsten ist.

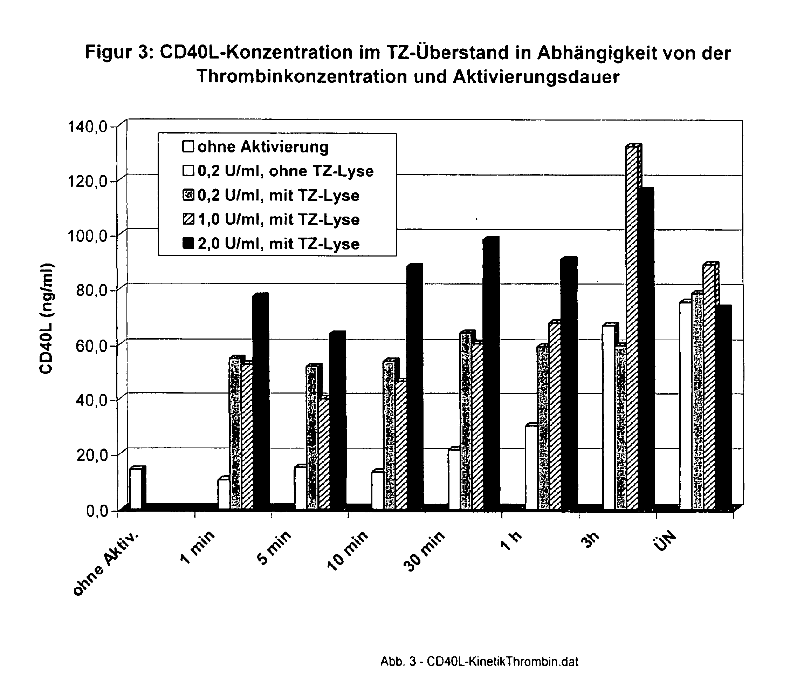

Figur 3 zeigt den Zusammenhang zwischen der Zugabe von Thrombin, einem Gefrierauftauvorgang (TZ-Lyse) und der Aktivierungsdauer.

### Beispiel 4

### Vergleich verschiedener Zentrifugationsgeschwindigkeiten

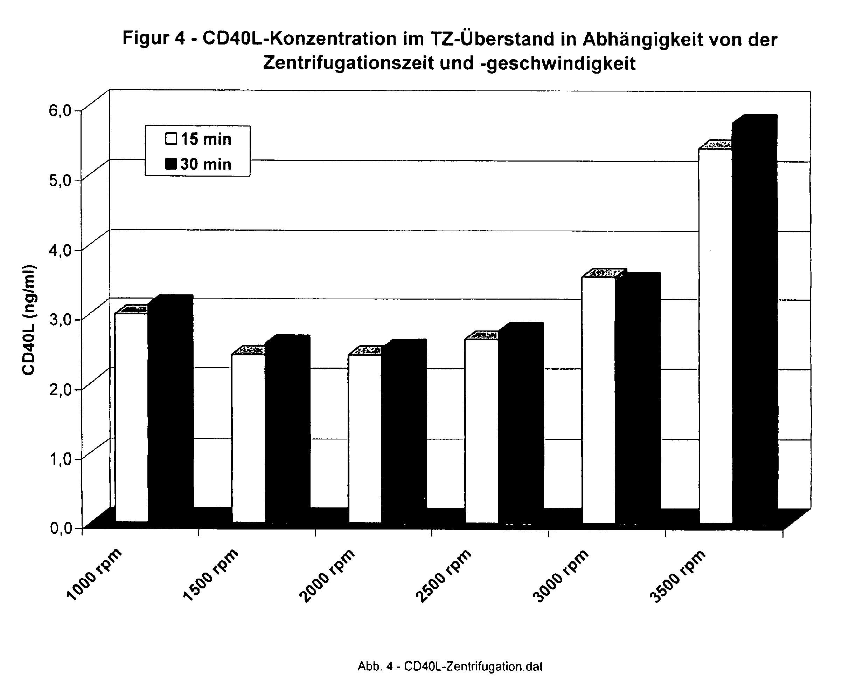

Der zu DC-Aktivierung generierte TZ-Überstand wurde wie oben beschrieben hergestellt ("bevorzugte Herstellungmethode"). Abweichend von der bevorzugten Herstellungsmethode wurden die TZ jedoch nicht aktiviert, sondern unterschiedlich stark und unterschiedlich lang zentrifugiert. Der Gehalt an CD40L wurde als Parameter der Qualität des TZ-Überstands bestimmt. Die Daten sind in Figur 4 dargestellt. Es zeigt sich, daß eine Zentrifugation bei 2000 x g (entspricht 3500 rpm) für 15-30 min hier die höchste Konzentration des TZ-Überstands bewirkt. Bei dem Versuch wurde eine Zentrifuge der Fa. Beckman (GS6-KR) mit dem Rotor GH3.8 verwendet. Die dadurch auf die Zellen ausgeübte Kraft (x g) errechnet sich daher nach folgender Formel: Zentrifugalkraft (RCF, Einheit g) = Radius (r) x Geschwindigkeit (ω)² / Schwerkraft (G). Für diese Zentrifuge ergibt dies RCF = 1,12 x r x (ω/1000)².

### Beispiel 5

### Vergleich verschiedener Konzentrationsverfahren des TZ-Überstands

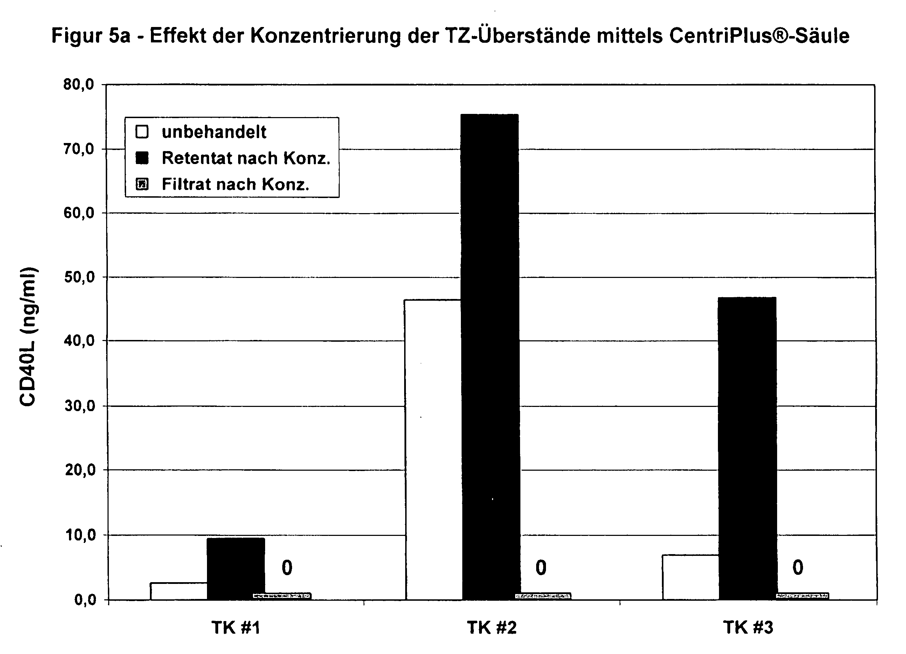

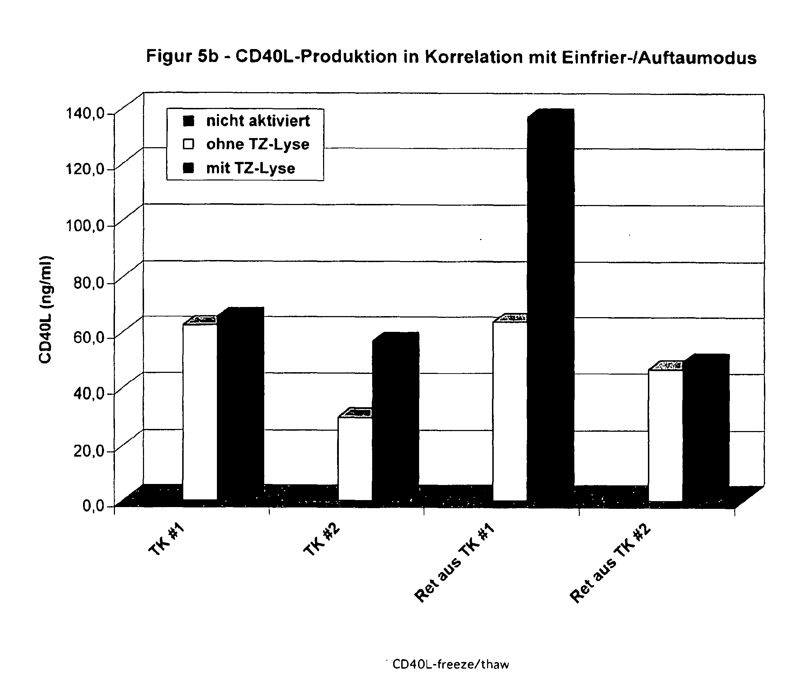

Der zu DC-Aktivierung generierte TZ-Überstand wurde wie oben beschrieben hergestellt ("bevorzugte Herstellungmethode"). Abweichend zu dieser wurden die TZ-Überstände entweder mit oder ohne Zelldisruption mittels Einfrieren und Auftauen hergestellt und anschließend entweder unbehandelt verwendet oder mit Hilfe von CentriPlus-Konzentratoren konzentriert. Der Gehalt an CD40L im TZ-Überstand bzw. im Filtrat oder Retentat der CentriPlus-Konzentratoren wurde als Parameter der Qualität des TZ-Überstands bestimmt. Es zeigt sich, dass die Konzentrierung des Überstands zu einer Erhöhung der CD40L-Konzentration führt und es zu keinem Verlust des CD40L kommt (Figur 5a). Zudem zeigt sich, daß die Lyse der TZ zumeist eine Erhöhung des CD40L-Gehalts bewirkt (Figur 5b).

### Beispiel 6

### Einfluss des Kulturmediums auf den CD40L-Gehalt des TZ-Überstands

Der zu DC-Aktivierung generierte TZ-Überstand wurde wie oben beschrieben hergestellt ("bevorzugte Herstellungmethode"). Abweichend zu dieser wurden die TZ-Überstände in unterschiedlichen Kulturmedien hergestellt. Der Gehalt an CD40L wurde als Parameter der Qualität des TZ-Überstands bestimmt. Es zeigt sich, daß die Anwesenheit von Serum für die Aktivität des TZ-Überstands nicht erforderlich ist, und daß die Verwendung von Ca⁺⁺/Mg⁺⁺-haltigem PBS die beste der hier getesteten Varianten darstellt.

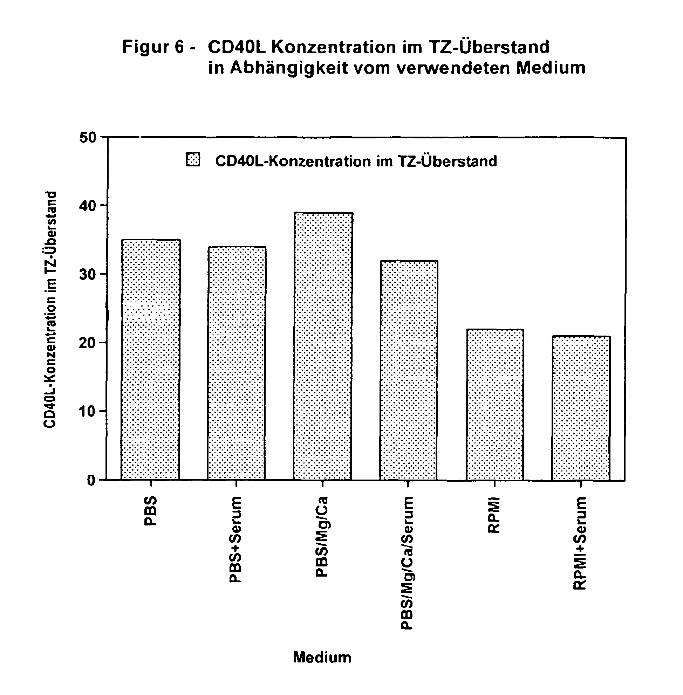

Figur 6 zeigt die Ergebnisse dieses Versuches.

### Beispiel 7

### Verklumpung der DC nach Zugabe des konditionierten Mediums

Bei Beobachtung mit dem Mikroskop sieht man, daß die DC ohne Zugabe von Aktivierungsreizen größtenteils als nicht-adhärenter monozellulärer Zellrasen in der in vitro Kultur wächst. Nach Zugabe von DC-Aktivatoren (hier als Kontrolle rekombinantes CD40L) bilden sich rasch grössere Zellklumpen, wobei die Stärke der Verklumpung zum Grad der DC-Aktivierung grob proportional ist. Es wurden TZ-Überstände in einer Konzentration zugegeben, die 20-124ng/ml CD40L enthalten. Man erkannte, daß bereits TZ-Überstände, die 20-25ng/ml CD40L enthalten, zu einer starken DC-Verklumpung führen, während dies für rekombinantes CD40L erst bei >0,5µg/ml der Fall ist. TZ-Überstände, die konzentrieter eingesetzt werden (entsprechend einer CD40L-Konzentration von >50 ng/ml), waren oft bereits toxisch für die DC.

Bei dem Experiment wurden PBMC wie oben beschrieben mittels Leukapherese gewonnen, plastikadhärente Zellen wurden in 1000 U/ml GM-CSF und 1000 U/ml IL-4 für 6 Tage inkubiert (in RPMI 1640 + 1 % autologem Serum). Anschließend wurde das Medium abpipettiert und erneuert, wobei zusätzlich verschiedene DC-Aktivatoren hinzugegeben wurden. Der zu DC-Aktivierung generierte TZ-Überstand wurde wie oben beschrieben hergestellt ("bevorzugte Herstellungmethode").

Die einzelnen Versuchsansätze wurden unter dem Mikroskop betrachtet und die Clustergrößen mit Hilfe eines Meßokulars gemessen. Von jedem experimentellen Ansatz wurden je 10 Cluster ausgezählt und der mittlere Durchmesser errechnet. Die Ergebnisse dieser Messung sind in der nachfolgenden Tabelle 1 zusammengefaßt. Die Tabelle 1 zeigt, daß die erfindungsgemäß eingesetzten Thrombozyten-Überstände erheblich aktiver sind als der Einsatz von rekombinant hergestelltem CD40 Ligand. Wenn allerdings zuviel Überstand zugegeben wurde, wurde ein toxischer Effekt für die dendritischen Zellen bemerkt (s. Thrombozyten-Überstand (entsprechend 100 ng/ml)).

**Tabelle 1: DC Clustergrösse in vitro nach Kultur mit TZ-Überstand im Vergleich zu recombinantem CD40L**

| **DC-Aktivierung** | **Durchmesser** | **SD** |
|---|---|---|
| TZ-Überstand (entspr. 25 ng/ml CD40L) | 125,0 | 74,2 |
| TZ-Überstand (entspr. 50 ng/ml CD40L) | 196,5 | 103,2 |
| TZ-Überstand (entspr. 100 ng/ml CD40L) | 48,5 | 33,1 |
| 500 ng/ml CD40L (recombinant) | 82,5 | 46,4 |
| 1000 ng/ml CD40L (recombinant) | 190,0 | 90,4 |
| keine DC-Aktivierung | 26,5 | 13,9 |

### Beispiel 8

### Expression von aktivierungsabhängigen Oberflächenmolekülen auf DC

DC wurden wie oben beschrieben mit TZ-Überstand oder auf andere Weise aktiviert und anschließend auf ihre Expression von aktivierungsabhängigen Oberflächenmolekülen durchflußzytometrisch überprüft. Die Expression der Moleküle CD80, CD83 und CD86 wird nach DC-Aktiverung stark induziert. Dies erfolgt insbesondere nach DC-Aktivierung mit IL-1 + IL-6 + TNFα + PGE₂, in etwas geringerem Maße jedoch auch durch Inkubation in TZ-Überstand.

PBMC wurden wie oben beschrieben mittels Leukapherese gewonnen, plastikadhärente Zellen wurden in 1000 U/ml GM-CSF und 1000 U/ml IL-4 für 6 Tage inkubiert (in RPMI 1640 + 1 % autologem Serum). Anschließend wurde das Medium abpipettiert und erneuert, wobei zusätzlich verschiedene DC-Aktivatoren hinzugegeben wurden. Der zu DC-Aktivierug generierte TZ-Überstand wurde wie oben beschrieben hergestellt ("bevorzugte Herstellungmethode"). 24 Std. nach Zugabe der DC-Aktivatoren wurden die Zellen mit Antikörpern gegen CD80, CD83 und CD86 markiert und im Durchflußzytometer (Fa. Coulter, Epics XL) analysiert. Die Ergebnisse dieses Versuchs wurden mit Hilfe der instrumenteneigenen Software ausgewertet.

Die erhaltenen Ergebnisse werden in den nachfolgenden Tabellen 2a - 2c zusammengefaßt.

**Tabelle 2a: Oberflächen-Phänotyp der DC nach Aktivierung**

| **CD80** | | |
|---|---|---|
| **DC-Aktivierung** | **% positiv** | **Fluoreszenzstärke** |
| IL1 + IL6 + TNFα + PGE₂ | 39.2 | 10.6 |
| IL1 + IL6 + TNFα + IFNγ | 17.2 | 5.9 |
| TZ-Überstand | 23.1 | 3.1 |
| TZ-Überstand + IFNγ | 39.1 | 6.2 |
| TZ-Überstand + TNFα + IFNγ | 32.4 | 6.9 |
| TZ-Überstand + IL1 + IL6 + TNFα + PGE₂ | 37.9 | 11.2 |
| keine DC-Aktivierung | 20.2 | 3.1 |

**Tabelle 2b: Oberflächen-Phänotyp der DC nach Aktivierung**

| **CD83** | | |
|---|---|---|
| **DC-Aktivierung** | **%positiv** | **Fluoreszenzstärke** |
| IL1 + IL6 + TNFα + PGE₂ | 43.5 | 20.5 |
| IL1 + IL6 + TNFα + IFNγ | 11.2 | 6.5 |
| TZ-Überstand | 16.9 | 7.6 |
| TZ-Überstand + IFNγ | 39.0 | 13.7 |
| TZ-Überstand + TNFα + IFNγ | 25.3 | 10.4 |
| TZ-Überstand + IL1 + IL6 + TNFα + PGE₂ | 35.2 | 13.2 |
| keine DC-Aktivierung | 8.3 | 4.3 |

**Tabelle 2c: Oberflächen-Phänotyp der DC nach Aktivierung**

| **CD86** | | |
|---|---|---|
| **DC-Aktivierung** | **%positiv** | **Fluoreszenzstärke** |
| IL1 + IL6 + TNF□ + PGE₂ | 42.0 | 49.8 |
| IL1 + IL6 + TNFα + IFNγ | 16.0 | 14.0 |
| TZ-Überstand | 44.9 | 10.9 |
| TZ-Überstand + IFNγ | 44.5 | 44.2 |
| TZ-Überstand + TNFα + IFNγ | 54.9 | 33.4 |
| TZ-Überstand + IL1 + 1L6 + TNFα + PGE₂ | 39.0 | 55.5 |
| keine DC-Aktivierung | 25.2 | 6.4 |

### Beispiel 9

### Antigenpräsentation durch DC in der allogenen gemischten Leukozytenreaktion (MLR)

DC wurden wie oben beschrieben mit TZ-Überstand oder auf andere Weise aktiviert und anschließend als APC in einer Standard-MLR eingesetzt. Es zeigt sich, daß bereits die Verwendung von TZ-Überständen, die 100 ng/ml CD40L enthalten, die gleiche allostimulatorische Kapazität haben, wie DC- die mit IL-1 + IL-6 + TNFα + PGE₂, oder mit IFNγ anstelle von PGE₂ stimuliert wurden. Die gleichzeitige Stimulation mit den verschiedenen Zytokinen plus TZ-Überstand führte zu einer geringgradig synergistischen Wirkung.

PBMC wurden wie oben beschrieben mittels Leukapherese gewonnen, plastikadhärente Zellen wurden in 1000 U/ml GM-CSF und 1000 U/ml IL-4 für 6 Tage inkubiert (in RPMI 1640 + 1 % autologem Serum). Anschließend wurde das Medium abpipettiert und erneuert, wobei zusätzlich verschiedene DC-Aktivatoren hinzugegeben wurden. Der zu DC-Aktivierung generierte TZ-Überstand wurde wie oben beschrieben hergestellt ("bevorzugte Herstellungmethode"). 24 Std, nach Zugabe der DC-Aktivatoren wurden die nichtadhärenten DC aus der Kultur entnommen, 2 x mit PBS gewaschen und mit allogenen T Zellen inkubiert. Die T Zellen wurden aus PBMC mittels Nylon-Wolle isoliert. 200.000 T Zellen wurden mit verschiedenen Konzentrationen der unterschiedlich aktivierten DC in 96-Loch Rundbodenplatten zusammen inkubiert. Nach 4 Tagen wurde 1 µCi ³H-Thymidin pro Loch für weitere 12 Std. hinzugegeben. Die Aufnahme des Thymidins dient als Meßparameter für die DC-induzierte Proliferation der allogenen T Zellen.

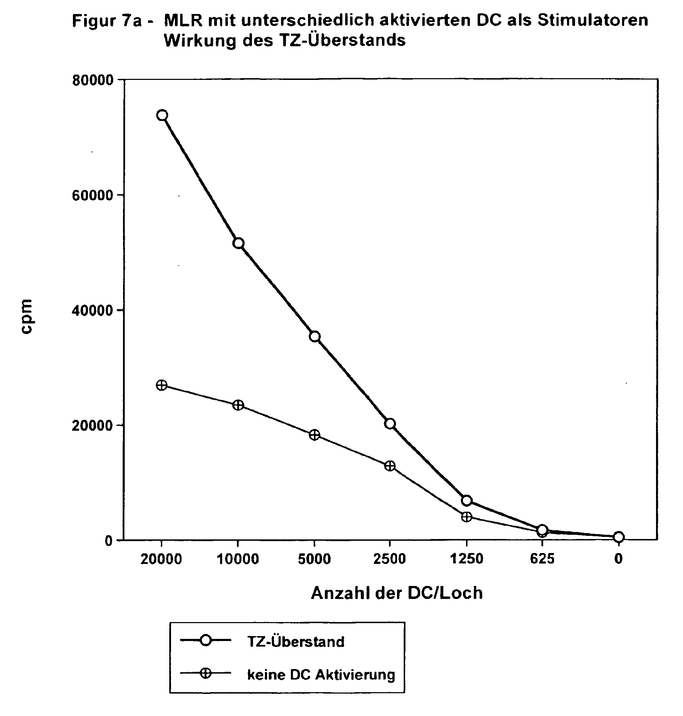

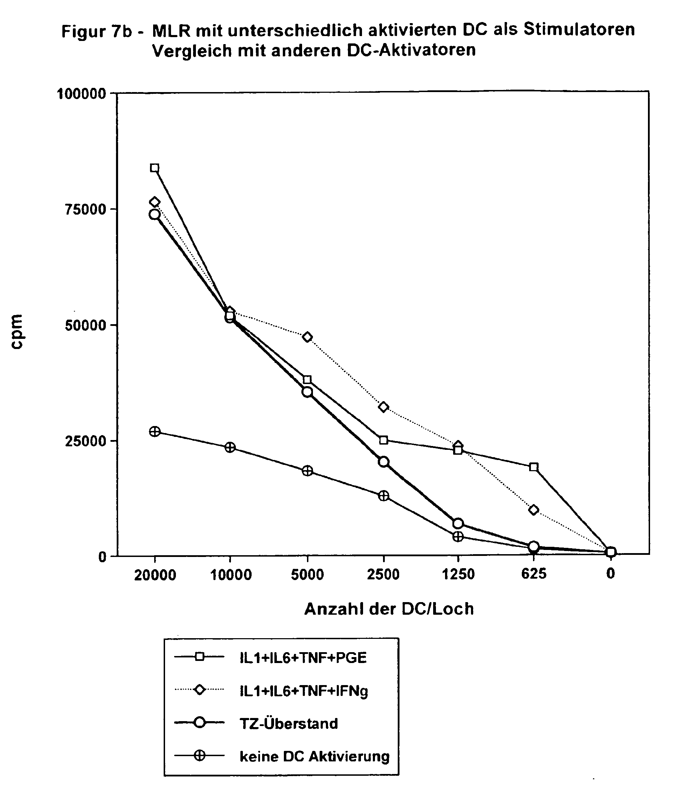

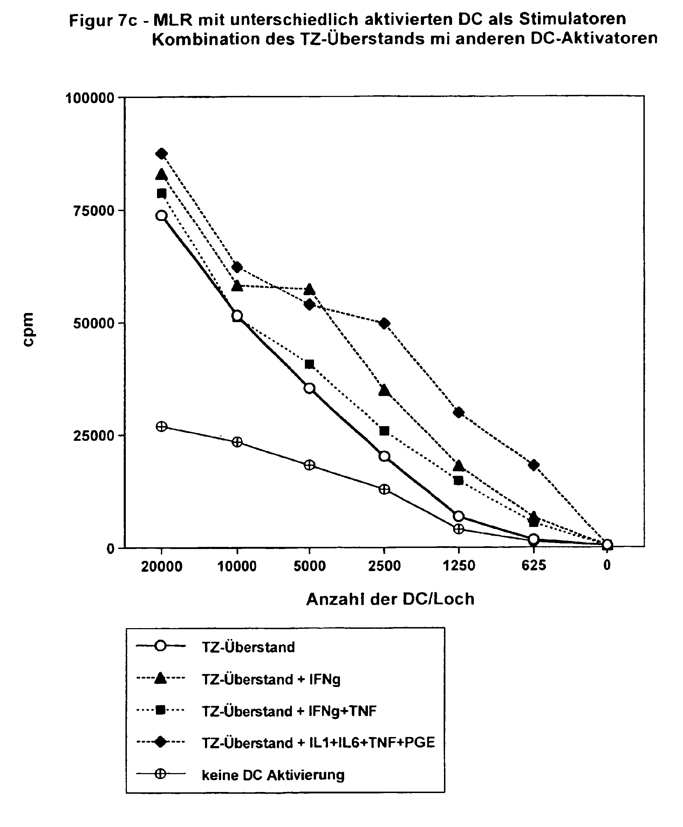

In Figur 7a ist gezeigt, welchen funktionellen Effekt der TZ-Überstand auf die Antigen-Präsentationskraft unreifer DC hat. Man erkennt deutlich, daß der TZ-Überstand eine starke Stimulation der Antigenpräsentation durch DC hat. In Abb. 7b wird dieser Effekt mit dem anderer DC-Aktivatoren verglichen. Man erkennt, daß die Wirkung des TZ-Überstands der anderer DC-Stimulatoren gleichwertig ist. In Abb. 7c ist gezeigt, daß der Effekt des TZ-Überstands partiell additiv zu dem der anderen DC-Aktivatoren ist. IL1, Interleukin-1, IL6, Interleukin-6, TNF, Tumor-Nekrose-Faktor α, PGE, Prostaglandin E, IFNg, Interferon γ.

### Beispiel 10

### DC-Ausbeute nach Aktivierung

DC wurden wie oben beschrieben mit TZ-Überstand oder auf andere Weise aktiviert und PBMC wurden wie oben beschrieben mittels Leukapherese gewonnen, plastikadhärente Zellen wurden in 1000 U/ml GM-CSF und 1000 U/ml IL-4 für 6 Tage inkubiert (in RPMI 1640 + 1 % autologem Serum). Anschließend wurde das Medium abpipettiert und erneuert, wobei zusätzlich verschiedene DC-Aktivatoren hinzugegeben wurden. Der zu DC-Aktivierung generierte TZ-Überstand wurde wie oben beschrieben hergestellt ("bevorzugte Herstellungmethode"). 24 Std. nach Zugabe der DC-Aktivatoren wurden die nichtadhärenten DC aus der Kultur entnommen, 2 x mit PBS gewaschen und gezählt. Die Anzahl der toten Zellen wurde mittels Propidiumjodid-Markierung durchflußzytometrisch bestimmt.

Die Ergebnisse des Versuchs sind in der nachfolgenden Tabelle 3 zusammengefaßt.

**Tabelle 3: DC-Ausbeute nach Aktivierung**

| **DC-Aktivierung** | **Zellzahl/Loch** ( | **% tote Zellen** |
|---|---|---|
| | in % der nicht aktivierten DC-Kultur) | |
| IL1 ± IL6 + TNFα + PGE₂ | 159 | 20 |
| IL1 + IL6 + TNFα + IFNγ | 104 | 27.7 |
| TZ-Überstand | 209 | 15,6 |
| TZ-Überstand + IFNγ | 132 | 18,2 |
| TZ-Überstand + TNFα + IFNγ | 110 | 18,5 |
| TZ-Überstand + IL1 + IL6 + TNFα + PGE₂ | 119 | 18,5 |
| keine DC-Aktivierung | 100 | 18,2 |

## Patentansprüche

1. In vitro-Verfahren zur Aktivierung von dendritischen Zellen, **dadurch gekennzeichnet, daß** dendritische Zellen mit einem Überstand von stimulierten Thrombozyten in Kontakt gebracht werden.

2. Verfahren nach Anspruch 1, **dadurch gekennzeichnet, daß** die dendritischen Zellen aus Monozyten des peripheren Bluts kultiviert werden.

3. Verfahren nach Anspruch 1, **dadurch gekennzeichnet, daß** die dendritischen Zellen aus anderen Vorläuferzellen, insbesondere Nabelschnurblut, kultiviert werden.

4. Verfahren nach einem der vorhergehenden Ansprüche, **dadurch gekennzeichnet, daß** die Vorläuferzellen in Gegenwart der Wachstumsfaktoren GM-CSF and IL-4 kultiviert werden.

5. Verfahren nach einem der vorhergehenden Ansprüche, **dadurch gekennzeichnet, daß** die Thrombozyten aus plättchenreichem Plasma hergestellt werden.

6. Verfahren nach Anspruch 5, **dadurch gekennzeichnet, daß** das plättchenreiche Plasma durch Leukapherese von Plasma hergestellt wird.

7. Verfahren nach einem der vorhergehenden Ansprüche, **dadurch gekennzeichnet, daß** die Thrombozyten **dadurch** aktiviert werden, daß sie in Kontakt mit einem Thrombozytenaktivator gebracht werden.

8. Verfahren nach Anspruch 7, **dadurch gekennzeichnet, daß** der Thrombozytenaktivator Thrombin oder ein Thrombinspaltprodukt ist.

9. Verfahren nach einem der vorhergehenden Ansprüche, **dadurch gekennzeichnet, daß** die stimulierten Thrombozyten autolog sind zu den dendritischen Zellen.

## Claims

1. In vitro method for activating dendritic cells, **characterized in that** dendritic cells are brought into contact with a supernatant of stimulated platelets.

2. Method according to Claim 1, **characterized in that** the dendritic cells are cultivated from peripheral blood monocytes.

3. Method according to Claim 1, **characterized in that** the dendritic cells are cultivated from other precursor cells, in particular umbilical cord blood.

4. Method according to any of the preceding claims, **characterized in that** the precursor cells are cultivated in the presence of the growth factors GM-CSF and IL-4.

5. Method according to any of the preceding claims, **characterized in that** the platelets are prepared from platelet-rich plasma.

6. Method according to Claim 5, **characterized in that** the platelet-rich plasma is prepared by leukapheresis of plasma.

7. Method according to any of the preceding claims, **characterized in that** the platelets are activated by bringing them into contact with a platelet activator.

8. Method according to Claim 7, **characterized in that** the platelet activator is thrombin or a thrombin cleavage product.

9. Method according to any of the preceding claims, **characterized in that** the stimulated platelets are autologous to the dendritic cells.

## Revendications

1. Procédé in vitro pour l'activation de cellules dendritiques, **caractérisé en ce que** les cellules dendritiques sont mises en contact avec un produit surnageant de thrombocytes stimulés.

2. Procédé selon la revendication 1, **caractérisé en ce que** les cellules dendritiques sont cultivées à partir de monocytes du sang périphérique.

3. Procédé selon la revendication 1, **caractérisé en ce que** les cellules dendritiques sont cultivées à partir d'autres cellules précurseurs, en particulier à partir du sang de cordon ombilical.

4. Procédé selon l'une quelconque des revendications précédentes, **caractérisé en ce que** les cellules précurseurs sont cultivées en présence des facteurs de croissance GM-CSF et IL-4.

5. Procédé selon l'une quelconque des revendications précédentes, **caractérisé en ce que** les thrombocytes sont préparés à partir de plasma riche en plaquettes.

6. Procédé selon la revendication 5, **caractérisé en ce que** le plasma riche en plaquettes est préparé par leucaphérèse du plasma.

7. Procédé selon l'une quelconque des revendications précédentes, **caractérisé en ce que** les thrombocytes sont activés par une mise en contact avec un activateur des thrombocytes.

8. Procédé selon la revendication 7, **caractérisé en ce que** l'activateur des thrombocytes et la thrombine ou un produit de dissociation de thrombine.

9. Procédé selon l'une quelconque des revendications précédentes, **caractérisé en ce que** les thrombocytes stimulés sont autologues vis-à-vis des cellules dendritiques.
